# FASCICULE DE BREVET EUROPEEN

(11) **EP 2 953 593 B1**
(45) Date de publication et mention de la délivrance du brevet: **12.10.2016**
(21) Numéro de dépôt: 14704614.8
(22) Date de dépôt: 27.01.2014
(51) Int. Cl.: A61F 5/44, A61M 1/00, A61F 5/445

(54) **DISPOSITIF PERMETTANT UN FLUX DE BOL ALIMENTAIRE ENTRE DEUX STOMIES**
VORRICHTUNG ZUR VERMITTLUNG EINES FLUSSES VON SPEISEBREI ZWISCHEN ZWEI STOMATA
DEVICE ALLOWING AN ALIMENTARY BOLUS FLOW BETWEEN TWO STOMAS

(30) Priorité: 05.02.2013 FR 1300229
(43) Date de publication de la demande: 16.12.2015
(73) Titulaire: Centre Hospitalier Regional Universitaire de Lille, 59037 Lille Cédex (FR)
(72) Inventeur: LOGIER, Régis, 59520 Marquette lez Lille (FR); SOZANSKI, Jean-Pierre, 59650 Villeneuve d'Ascq (FR); NZAMUSHE LEPAN MABLA, Jean-Robert, 1420 Braine l'Alleud (BE)
(74) Mandataire: Schwalek, Valérie
(86) Numéro de dépôt international: PCT/FR2014/050148
(87) Numéro de publication internationale: WO 2014/122378

(56) Documents cités:
- WO-A2-2009/046997
- US-A1- 2001 023 337
- US-A1- 2004 220 516
- US-A1- 2008 039 809

## Description

La présente invention concerne un dispositif permettant un flux de bol alimentaire.

Pour traiter des pathologies telles que la diverticulite aiguë, le cancer du rectum, les traumatismes intestinaux ou les maladies intestinales inflammatoires, on a fréquemment recourt à l'ablation d'une partie de l'intestin et à la pratique de stomies. On vient ainsi suturer les extrémités de l'intestin formées par l'ablation d'une partie de ce dernier, au niveau de la peau de l'abdomen du patient. Les deux extrémités sont suturées ouvertes, de manière à ce que l'intérieur de l'intestin soit accessible au niveau de l'abdomen du patient. La stomie désigne donc, au sens de la présente invention, l'extrémité ouverte de l'intestin qui affleure au niveau de la peau de l'abdomen du patient. Cette approche thérapeutique qui peut être temporaire ou permanente crée de nombreuses contraintes en termes de qualité de vie et de fonctionnement du système digestif.

Dans les colostomies (stomies du côlon) et iléostomies (stomies de l'intestin grêle), la fonction intestinale normale est interrompue et le bol alimentaire traverse la paroi abdominale à travers la stomie. Le bol alimentaire vient alors remplir une poche de stomie qui doit être vidée régulièrement. Bien que tous les efforts soient faits pour préserver l'intégrité de l'intestin et des tissus et pour traiter et réduire la douleur et l'inconfort des patients, dans de nombreux cas, la stomie conduit à une intensification de la détresse et de la souffrance des patients. De plus, certains patients iléostomisés nécessitent une alimentation parentérale (i.e. par voie intraveineuse) du fait de la trop faible absorption des nutriments par voie orale résultant de la trop faible longueur d'intestin traversée par le bol alimentaire. L'alimentation parentérale au long terme peut s'accompagner d'effets secondaires tels que des complications infectieuses et/ou des perturbations hépatiques. Elle nécessite, de plus, une hospitalisation au long cours en hôpital ou au domicile, occasionnant des frais élevés. A cela s'ajoute une qualité de vie altérée entraînant une sédentarité et des soins de stomie itératifs.

Par ailleurs, lorsqu'il n'y a plus de risque d'infection du péritoine, les deux portions de l'intestin sont reconnectées l'une à l'autre ; la portion aval qui n'a pas été traversée par le bol alimentaire durant toute la durée d'interruption du transit s'avère être altérée, ce qui complique la fin de la convalescence et la reprise d'un transit intestinal normal permettant une absorption normale des nutriments.

Dans de telles circonstances, il est important d'identifier des moyens efficaces pour améliorer la qualité de vie des patients stomisés.

Le document WO2009046997A2 décrit un dispositif de mise en circulation du bol alimentaire destiné à être implanté dans le corps du patient pour permettre la sortie du bol au niveau d'une stomie ou pour permettre la circulation du bol alimentaire entre deux portions d'intestin raccordées par le dispositif implanté dans le corps du patient. Ce type de dispositif génère des problèmes d'infection en cas de fuite de bol alimentaire dans le corps du patient. L'implantation du dispositif nécessite une intervention chirurgicale qui n'est pas sans danger sur un patient déjà affaibli par une pathologie.

Le préambule de la revendication indépendante 1 est fondé sur ce document.

Un but de la présente invention est de proposer un nouveau dispositif qui permet d'améliorer la qualité de vie des patients qui ont subi une intervention chirurgicale et sur lesquels on a pratiqué des stomies lors de ladite intervention.

Dans ce but, la présente invention propose un dispositif permettant de créer un flux de bol alimentaire comme défini dans la revendication 1, comportant des moyens formant pompe qui présentent une ouverture d'aspiration et une ouverture de refoulement et qui sont adaptés pour aspirer du bol alimentaire à travers ladite ouverture d'aspiration et le refouler à travers ladite ouverture de refoulement. Selon la présente invention, de manière caractéristique, le dispositif comporte en outre, des premiers moyens de connexion étanche aptes à relier ladite ouverture d'aspiration à une stomie amont, située sur la paroi abdominale d'un patient ; des deuxièmes moyens de connexion étanche aptes à relier ladite ouverture de refoulement à une stomie aval, disposée sur la paroi abdominale dudit patient ; lesdits moyens formant pompe sont actionnables par un utilisateur et sont aptes à être montés sur le corps d'un patient, notamment au niveau de son abdomen, moyennant quoi lorsque le dispositif est monté sur l'abdomen d'un patient, du bol alimentaire peut circuler de ladite stomie amont vers ladite stomie aval, à l'extérieur de l'abdomen dudit patient.

Des modes de réalisation particuliers de l'invention sont définis dans les revendications dépendantes.

La circulation du bol alimentaire dans la totalité des intestins du patient permet de conserver toute sa fonctionnalité à la portion de l'intestin situé en aval des stomies. A terme, toute alimentation parentérale peut être supprimée du fait d'une meilleure absorption des nutriments. Le dispositif étant portable, il ne gêne pas les mouvements du patient.

Dans le cadre de la présente invention, les termes « bol alimentaire » font référence au bol alimentaire intestinal, c'est-à-dire au bol alimentaire contenu dans les intestins du patient.

La pose du dispositif de l'invention ne nécessite aucune intervention chirurgicale et utilise les stomies formées. L'utilisation du dispositif de l'invention ne génère aucun risque d'infection puisque le bol alimentaire circule à l'extérieur du corps du patient lorsqu'il ne circule pas dans une portion d'intestin. Les endroits de fuite possible sont donc situés à l'extérieur du corps du patient.

Les moyens formant pompe peuvent, par exemple, être fixés sur une ceinture, sur une bandoulière, sur une combinaison des deux, sur une paire de bretelles ou sur tout autre élément susceptible d'être porté par un utilisateur-patient ou accroché à son corps. Ils sont ainsi aptes à être montés sur le corps d'un patient.

Selon un mode de réalisation particulier des moyens de connexion étanche, lesdits premiers moyens de connexion étanche et/ou lesdits deuxièmes moyens de connexion étanche comportent un socle sur lequel est monté un collecteur de bol alimentaire, ledit socle présente une première face apte à être fixée de manière étanche sur la peau d'un patient, une deuxième face qui comporte des moyens de montage dudit collecteur et au moins un passage ménagé dans ledit socle et débouchant dans ledit collecteur.

Le socle peut, par exemple, être collé sur la peau du patient. Le mode de fixation du socle sur la peau de l'abdomen du patient n'est pas limité selon l'invention. Le passage ménagé dans le socle est destiné à être disposé en regard d'une stomie. Aussi, lorsque les premiers et les deuxièmes moyens de connexion étanche sont distincts et disposés chacun au niveau d'une seule stomie, chacun ne comportent qu'un passage débouchant dans le collecteur.

Avantageusement, les deuxièmes moyens de connexion étanche comportent un tube de refoulement relié à ladite sortie de refoulement et conformé pour être introduit dans l'intestin d'un patient au niveau de ladite stomie aval. Ceci permet d'éviter toute fuite et tout reflux vers la stomie aval. Lorsque les deuxièmes moyens de connexion étanche comporte un socle dans lequel est ménagé un passage, le tube de refoulement traverse le passage et s'étend au-delà du socle, dans la direction opposée au collecteur.

Une sonde dite de Pezzer peut être avantageusement utilisée comme tube de refoulement.

Selon un mode de réalisation particulier des moyens de connexion étanche, lesdits premiers moyens de connexion étanche et lesdits deuxièmes moyens de connexion étanche comportent un socle commun et un collecteur commun. Ce mode de réalisation particulier est bien adapté pour des stomies dites rapprochées, qui sont disposées l'une à côté de l'autre. Dans ce cas le socle comporte deux passages qui sont agencés de manière à être disposés chacun en regard d'une stomie.

Les moyens formant pompe ne sont pas limités selon l'invention. Selon un mode de réalisation particulier des moyens formant pompe, lesdits moyens formant pompe comportent au moins un élément déformable et des moyens de déformation dudit élément déformable, ledit élément déformable définit au moins partiellement un espace communiquant avec lesdits premiers moyens de connexion étanche au niveau de ladite ouverture d'aspiration et communiquant avec lesdits deuxièmes moyens de connexion étanche, au niveau de ladite ouverture de refoulement, les déformations dudit élément déformable modifient le volume dudit espace, moyennant quoi lorsque ledit volume dudit espace diminue, le bol alimentaire contenu dans ledit espace est expulsé dans lesdits deuxième moyens de connexion étanche et lorsque ledit volume dudit espace augmente du bol alimentaire provenant desdits premiers moyens de connexion étanche est aspiré dans ledit espace.

Selon un mode de réalisation, ledit élément déformable est une membrane, lesdits moyens formant pompe comportent un carter dont l'espace interne est divisé en une première et une deuxième chambre par ladite membrane, ladite première chambre communique avec lesdits premiers moyens de connexion étanche et avec lesdits deuxièmes moyens de connexion étanche, lesdits moyens formant pompe comportent, en outre, des moyens d'injection/aspiration d'un fluide qui sont reliés à ladite deuxième chambre et qui sont adaptés pour faire varier la pression dudit fluide dans ladite deuxième chambre, moyennant quoi lesdits moyens d'injection/aspiration d'un fluide font varier la courbure de ladite membrane déformable ce qui permet de faire varier le volume de la première chambre et d'aspirer du bol alimentaire dans ladite première chambre ou d'expulser du bol alimentaire hors de ladite première chambre.

Avantageusement, le dispositif comporte des premiers moyens formant clapet disposés pour éviter le reflux du bol alimentaire aspiré par lesdits moyens formant pompe vers lesdits premiers moyens de connexion étanche et/ou des deuxièmes moyens formant clapet pour éviter le reflux du bol alimentaire refoulé par lesdits moyens formant pompe vers lesdits deuxième moyens de connexion étanche.

Les premiers et deuxièmes moyens formant clapet ne sont pas limités selon l'invention. Il peut s'agir de tout type de clapet existant, des clapets de type bec de canard, des valves ou autre.

Les moyens formant pompe peuvent être activés au moyen d'un interrupteur situé sur les moyens formant pompe ou qui est apte à être monté sur le corps du patient.

Avantageusement, le dispositif comporte des moyens de commande des moyens formant pompe qui sont distincts des moyens formant pompe. Ces moyens de commande peuvent être détachés du corps du patient et actionnés à distance (par IR ou autre). Les moyens de commande peuvent être une télécommande spécialement dédiée aux moyens formant pompe du dispositif de l'invention. Ils peuvent également comprendre une tablette, un téléphone portable ou tout autre élément programmable et susceptible d'interagir avec les moyens formant pompe.

Avantageusement, le dispositif comporte des moyens de commande desdits moyens formant pompe, lesdits moyens de commande comportent une horloge interne temps-réel adaptée pour actionner lesdits moyens formant pompe au bout d'une durée de repos donnée, choisie par l'utilisateur et des moyens de programmation de ladite horloge interne temps réel qui permettent de choisir ladite durée de repos.

L'action de pompage automatique au bout de la période de repos permet également d'amorcer le transit dans la partie de l'intestin situé en amont de la stomie.

Selon un mode de réalisation des moyens de commande, lesdits moyens de commande comportent, en outre :
- des moyens de détection de la présence du bol alimentaire en aval desdits premiers moyens de connexion étanche et/ou en amont desdits deuxième moyens de connexion étanche qui sont adaptés pour mesurer un paramètre variable en fonction de la présence ou non de bol alimentaire et actionner lesdits moyens formant pompe lorsque la valeur dudit paramètre correspond à la présence de bol alimentaire dans ledit dispositif ;
- des moyens de déclenchement desdits moyens de détection qui actionnent lesdits moyens de détection au bout d'une durée test choisie par l'utilisateur ; et
- des moyens de réglage de ladite durée test.

Lesdits moyens de détection peuvent, par exemple, comporter au moins un capteur choisi parmi les capteurs capacitifs, les capteurs optiques, les capteurs de température et les capteurs de pression.

Avantageusement, les moyens de commande comportent, en outre, des moyens de déclenchement automatique desdits moyens formant pompe au bout d'une durée de sécurité programmable par le patient/ utilisateur. Le dispositif de l'invention peut ainsi être utilisé en cas de défaillance des moyens de détection de la présence du bol alimentaire, sans intervention spécifique de la part du patient.

La présente invention peut être mise en oeuvre dans un procédé de mise en circulation d'un bol alimentaire entre une stomie amont pratiquée dans l'abdomen d'un patient et une stomie aval, selon lequel on aspire du bol alimentaire contenu dans l'intestin du patient en amont de ladite stomie amont et on refoule ensuite ledit bol alimentaire aspiré dans l'intestin dudit patient au niveau de ladite stomie aval ou en aval de ladite stomie aval. Ce procédé peut être utilisé pour améliorer l'alimentation du patient ce qui contribue à sa convalescence après une intervention qui a nécessité la pratique de stomies. Ce procédé permet de réduire la durée de la période pendant laquelle le patient est stomisé, cette période précédant l'éventuelle opération de raccordement des deux portions intestinales.

Avantageusement, on détecte la présence de bol alimentaire au niveau de ladite stomie amont ou entre ladite stomie amont et ladite stomie aval avant d'aspirer le bol alimentaire contenu dans l'intestin en amont de ladite stomie amont.

A cet effet, un produit programme d'ordinateur comporte des instructions qui lorsqu'il est mis en oeuvre sur un calculateur associé à un dispositif selon l'invention met en oeuvre l'un des procédés de mise en circulation d'un bol alimentaire précités.

La présente invention, ses caractéristiques et les différents avantages qu'elle procure apparaitront plus clairement à la lecture de la description détaillée qui suit de deux modes de réalisation, présentés à titre d'exemples explicatifs et non limitatifs, en référence aux figures annexées sur lesquelles :
- la Fig. 1 représente un schéma de principe d'un premier mode de réalisation de l'invention, monté sur l'abdomen d'un patient présentant deux stomies rapprochées ;
- la Fig. 2 représente de manière schématique une vue du dessus d'un mode de réalisation particulier des premiers moyens de connexion étanche, avec une coupe partielle selon un plan horizontal ;
- la Fig. 3 représente de manière schématique une vue partiellement en coupe transversale d'une variante de réalisation du premier mode de réalisation ;
- la Fig. 4 représente un schéma de principe d'un second mode de réalisation de l'invention monté sur un patient et selon une coupe transversale parallèlement à un plan vertical ;
- la Fig. 5 représente une vue en coupe transversale selon l'axe longitudinal de l'intestin, d'une partie des moyens formant pompe et d'une variante de réalisation des premiers et deuxième moyens de connexion étanche du deuxième mode de réalisation ; et
- la Fig. 6 représente une vue en coupe transversale partielle d'une variante de réalisation du second mode de réalisation représenté sur la Fig. 5.

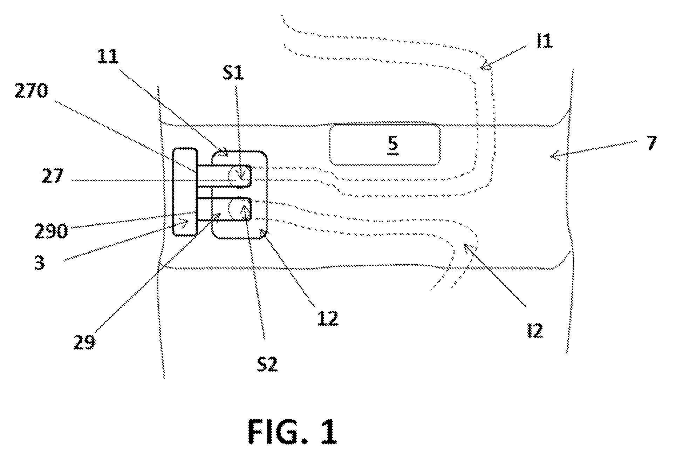

En référence à la Fig. 1, l'abdomen du patient présente une stomie amont S1 et une stomie aval S2. Ces deux stomies sont adjacentes et sont appelées stomies rapprochées. En fonction du type d'intervention, les stomies peuvent également être plus éloignées, et par exemple, disposées chacune sur un flanc du patient. La stomie amont S1 communique avec la portion amont I1 de l'intestin qui fait suite à l'estomac (non représenté). La stomie aval S2 communique avec la portion aval I2 de l'intestin qui se poursuit jusqu'à l'anus (non représenté). Les deux portions I1 et I2 de l'intestin sont représentées en traits pointillés.

Sur la Fig. 1, les premiers moyens de connexion étanche 11 sont montés sur la stomie amont S1, tandis que les deuxièmes moyens de connexion étanche 12 sont montés sur la stomie aval S2. Les premiers et deuxième moyens de connexion étanches 11 et 12 sont connectés à des moyens formant pompe 3 qui sont commandés par des moyens de commande 5. Les premiers moyens de connexion étanche 11 sont reliés aux moyens formant pompe 3 au moyen du conduit d'aspiration 27 qui débouche dans les moyens formant pompe 3, au niveau d'une ouverture d'aspiration 270. Les deuxième moyens de connexion étanche 12 sont reliés au moyens formant pompe 3 par le conduit de refoulement 29. Le conduit de refoulement 29 débouche dans les moyens formant pompe 3 au niveau d'une ouverture de refoulement 290. Les premiers et deuxièmes moyens de connexion étanche 11 et 12 et les moyens formant pompe 3 sont montés sur une ceinture 7 qui entoure l'abdomen du patient. Le dispositif de l'invention est ainsi portatif et ne gêne en rien la mobilité du patient. Les moyens de commande 5 sont également, dans le mode de réalisation ici représenté, montés sur la ceinture 7. Ils peuvent néanmoins, selon la présente invention être démontés de la ceinture ou totalement distincts de cette dernière. Ils peuvent par exemple, comporter une tablette tactile existante qui fonctionne avec un produit programme d'ordinateur spécifique.

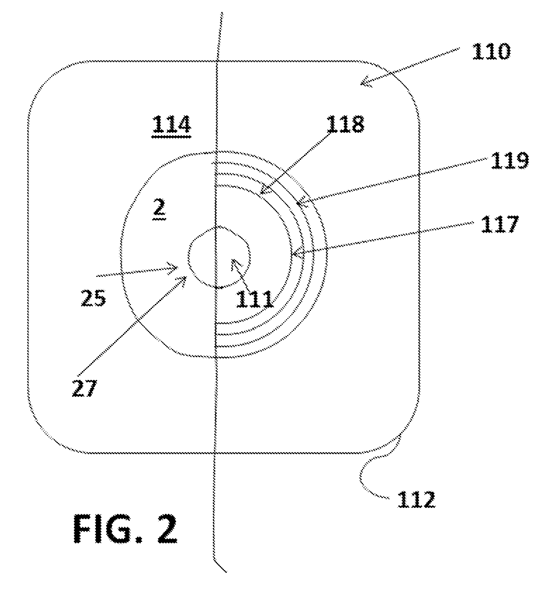

En référence à la Fig. 2, un mode de réalisation particulier des premiers moyens de connexion étanche 11 va maintenant être décrit. Les premiers moyens de connexion étanche 11 comportent un socle 110 qui est une feuille souple. Ce socle 110 présente une face inférieure 112 qui est revêtue d'une colle spéciale permettant la fixation du socle sur la peau d'un patient. La face supérieure 114 du socle 110 comporte une pièce de fixation 117 qui est rapportée sur le socle 110. Cette pièce de fixation comporte une collerette 119, sensiblement perpendiculaire au socle 110, lequel est sensiblement plan. Une gorge d'étanchéité 118 entoure la collerette 119. La collerette 119 et la gorge 118 entourent un passage 111 ménagé dans le socle 110. La pièce de fixation 117 entoure le passage 111. Le passage 111 est entouré par la gorge 118 qui est elle-même entourée par la collerette 119. Lorsque les premiers moyens de connexion étanche 11 sont montés sur l'abdomen du patient, le passage 111 est disposé au niveau de la stomie amont S1 de manière à permettre le passage du bol alimentaire à travers le passage 111.

Comme représenté sur la Fig. 2, les premiers moyens de connexion étanche 11 comportent également un collecteur 2. Le collecteur 2 est un boîtier qui est apte à être monté de manière étanche sur la pièce de fixation 117 et qui présente une ouverture apte à être reliée à un tube de refoulement, comme ultérieurement expliqué.

Les deuxièmes moyens de connexion étanche 12 peuvent être identiques aux premiers moyens 11 précités, à l'exception du conduit d'aspiration 27 qui est remplacé par le conduit de refoulement 29 relié à la sortie de refoulement des moyens formant pompe.

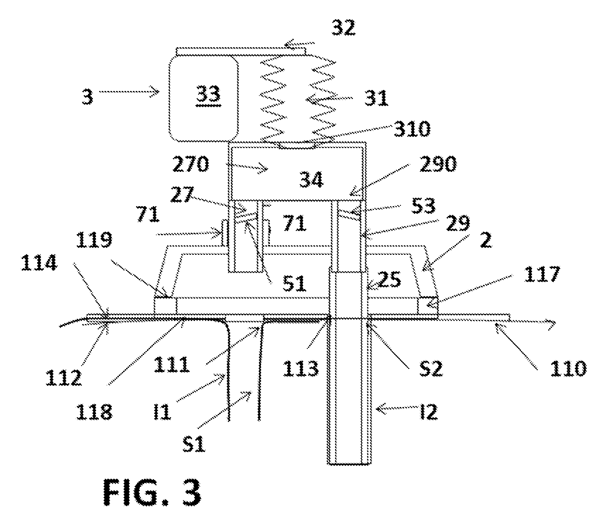

En référence à la Fig. 3, une variante de réalisation du premier mode de réalisation de l'invention va maintenant être décrite. Dans cette variante de réalisation particulière, les premiers moyens de connexion 11 et les deuxièmes moyens de connexion 12 comportent un même socle 110 et un même collecteur 2. Le socle 110 comporte un premier passage 111 et un second passage 113 qui sont disposés côte-à-côte. Le socle 110 est collé sur la peau de l'abdomen d'un patient. Le premier passage 111 est disposé en regard de la stomie amont S1, tandis que le second passage 113 est disposé en regard de la stomie aval S2. Le collecteur 2 est monté sur le socle 110 au moyen de la pièce de fixation 117. Le conduit d'aspiration 27 débouche dans le collecteur 2 sensiblement au-dessus de la stomie amont S1. L'autre extrémité du conduit d'aspiration 27 débouche dans un conduit de liaison 34, au niveau d'une ouverture d'aspiration 270. Le collecteur 2 comporte également une deuxième perforation qui est traversée par le conduit de refoulement 29, lequel est disposé au-dessus de la stomie aval S2. Le conduit de refoulement 29 débouche également dans le conduit de liaison 34, au niveau d'une ouverture de refoulement 290. L'autre extrémité du conduit de refoulement 29 se trouve dans le collecteur 2, au-dessus de la stomie aval S2 et est reliée à un tube de refoulement 25 qui se prolonge à travers le deuxième passage 113 et s'étend au-delà du socle 110. Sur la Fig. 3, le tube de refoulement 25 s'étend dans la portion aval I2 de l'intestin, en aval de la stomie aval S2. En référence à la Fig. 3, les moyens formant pompe 3 comportent un soufflet 31 actionné par un moteur 33 au moyen d'un bras 32. Le moteur 33 est dans le cas présent un moteur linéaire. Un système de bielle manivelle associé à un moteur rotatif peut également être utilisé. Le soufflet 31 comporte une ouverture 310 qui débouche dans le conduit de liaison 34, entre l'ouverture d'aspiration 270 et l'ouverture de refoulement 290. Un clapet anti-retour 51, du type bec de canard, par exemple, équipe le conduit d'aspiration 27 de manière à éviter tout reflux du bol alimentaire aspiré dans le conduit de liaison 27 vers le collecteur 2. De même, un clapet anti-retour 53, du type bec de canard, équipe le conduit de refoulement 29, de manière à éviter tout reflux du bol alimentaire situé dans le tube de refoulement 25 vers le conduit de liaison 34.

Des moyens de détection de la présence de bol alimentaire en amont des moyens formant pompe équipent le conduit d'aspiration 27, entre sa sortie du collecteur 2 et l'ouverture d'aspiration 270. Ces moyens comportent un capteur capacitif 71 qui permet de mesure la permittivité du milieu contenu dans le conduit d'aspiration 27. Le capteur capacitif 71 est formé à partir de deux plaques de dimension de l'ordre de 15 mm par 10 mm placées autour du conduit d'aspiration 27. Ce capteur capacitif 71 est équivalent à un condensateur plan. La valeur de la capacité de ce condensateur dépend du milieu donc la présence du bol alimentaire dans le conduit d'aspiration 27. La valeur de cette capacité est de l'ordre de quelques picofarads lorsque le conduit d'aspiration 27 est rempli d'air et s'élève à quelques centièmes de picofarads, lorsque du bol alimentaire se trouve dans le conduit d'aspiration 27.

Le capteur capacitif 71 peut être remplacé par un capteur optique formé d'une diode LED dans le domaine IR ou visible et d'une photodiode placée en regard de la LED, le conduit d'aspiration 27 séparant la LED et la photodiode. La réponse de la photodiode est quasi proportionnelle à l'intensité lumineuse émise par la LED. Ainsi, lorsque du bol alimentaire se trouve dans le conduit d'aspiration 27, la réponse de la photodiode diminue du fait de l'absorption de la lumière émise par la LED par le bol alimentaire.

Un capteur de température peut également être utilisé. Une élévation de température dans le conduit d'aspiration indiquant la présence de bol alimentaire.

Un capteur choisi parmi les divers types de capteur précités peut aussi équiper le conduit de refoulement 29 en plus du capteur équipant le conduit d'aspiration 27 ou en remplacement de ce capteur.

Les moyens de commande 5 comportent, selon un mode de réalisation, un microcontrôleur associé à un logiciel spécifique qui est dédié à la gestion des différents capteurs et du pilotage du moteur 33 des moyens formant pompe 3. Ils comportent également, un module d'affichage LCD, une interface touche de fonctions et de LED de signalisation, une mémoire non volatile pour le stockage des informations pendant toute la période de soin (plusieurs semaines), des moyens de conditionnement des signaux des capteurs, un module Bluetooth® pour la lecture et l'écriture à distance des informations relatives aux mesures des capteurs et la programmation spécifique en fonction des besoins du patient.

Le fonctionnement de ce premier mode de réalisation va être décrit en référence aux figures 1 et 3.

On effectue tout d'abord un étalonnage du dispositif, avant son fonctionnement. Le capteur 71 mesure la capacité de l'air remplissant le conduit d'aspiration 27. Cette valeur correspond à la valeur de référence du dispositif « à vide », c'est-à-dire ne contenant pas de bol alimentaire.

Le capteur 71 mesure ensuite régulièrement la capacité du milieu contenu dans le conduit d'aspiration 27. La durée entre deux mesures de capacité correspond à une durée test, réglable par l'utilisateur par le biais des moyens de commande 5. Lorsque du fait des contractions de la portion amont I1 de l'intestin (ou du pompage es moyens formant pompe 3), du bol alimentaire sort par la stomie amont S1, le bol alimentaire pénètre dans le collecteur 2 et parvient dans la section du conduit d'aspiration 27 située au niveau du capteur 71. Le capteur 71 effectue ses mesures régulièrement, deux mesures consécutives étant espacées de la durée de test. Une de ces mesures est effectuée lorsque le bol alimentaire est dans le conduit d'aspiration 27. Le capteur 71 mesure la capacité du milieu remplissant le conduit d'aspiration, la compare avec la valeur de référence et détecte la présence de bol alimentaire. Il actionne alors les moyens formant pompe 3. Le moteur 33 comprime alors le soufflet 31 puis le détend aspirant ainsi le bol alimentaire depuis le collecteur 2 dans le soufflet 31 à travers l'ouverture 310. Lorsque le soufflet 31 est déplié, il contient du bol alimentaire, le moteur 33 comprime à nouveau le soufflet 31 et le bol alimentaire est alors expulsé dans le conduit de liaison 34 et passe à travers le tube de refoulement 25. Le bol alimentaire est donc ainsi injecté dans la portion aval I2 de l'intestin.

Les moyens de détection de la présence de bol alimentaire peuvent comprendre deux capteurs qui réalisent chacun la mesure d'un paramètre différent. Si les deux capteurs indiquent la présence de bol alimentaire, ils déclenchent les moyens formant pompe pour aspirer le bol alimentaire contenu dans le conduit d'aspiration.

De préférence, le dispositif comporte également des moyens de déclenchement de sécurité qui actionnent systématiquement les moyens formant pompe au bout d'une durée de sécurité réglable par l'utilisateur via les moyens de commande.

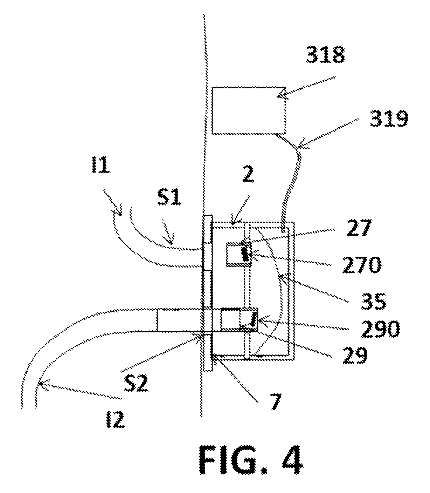

Un deuxième mode de réalisation va maintenant être décrit en référence aux Fig. 4 à 6. Les éléments en commun avec le premier mode de réalisation sont référencés à l'identique.

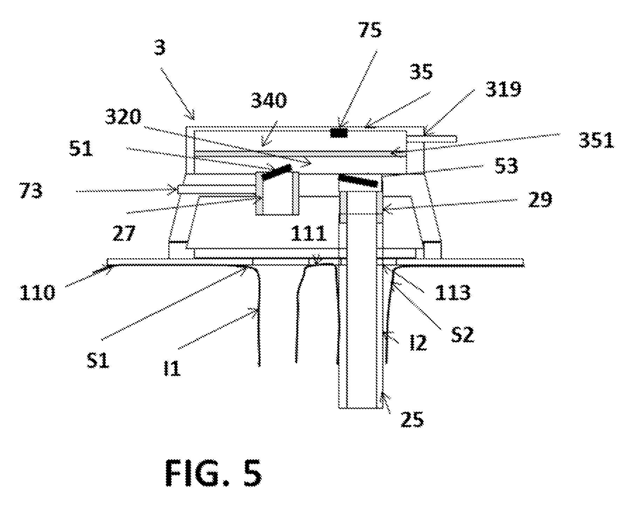

En référence à la Fig. 4, le dispositif de l'invention comporte des premiers moyens de connexion étanche 11 qui forment également les deuxièmes moyens de connexion étanche. Sur la Fig. 4, les premiers moyens de connexion étanche 11 sont montés sur l'abdomen du patient comme en référence au premier mode de réalisation. La ceinture 5 supporte une pompe à air 318 et les moyens de commande 5. Le collecteur 2 est surmonté d'une pompe à membrane contenue dans un carter 35 qui sera décrit plus en détail en référence à la Fig. 5. La pompe à membrane est reliée à la pompe à air 318 via le tube de raccordement 319.

Dans ce second mode de réalisation, les premiers et deuxièmes moyens de connexion étanche comportent un même support 110 et un même collecteur 2. Comme représenté sur la Fig. 5, le collecteur 2 est surmonté d'un carter 35. Le carter 35 forme un logement étanche au-dessus du collecteur 2. Le carter 35 comporte une membrane 351 déformable qui sépare le logement en deux chambres, une première chambre 320 de réception du bol alimentaire et une deuxième chambre dite d'actionnement 340. Le conduit d'aspiration 27 et le conduit de refoulement 29 débouchent dans la première chambre 320. Les conduits précités sont ménagés dans l'épaisseur de la paroi supérieure du collecteur 2. La membrane 351 est sensiblement parallèle à la face supérieure du collecteur 2 qui forme le fond du logement défini par le carter 35 et donc sensiblement perpendiculaire aux conduits 27 et 29. La deuxième chambre 340 qui se situe, sur la Fig. 5, au-dessus de la première 320, est reliée à une pompe à air (non représentée sur la Fig. 5) au moyen d'un tube de raccordement 319.

En référence à la Fig. 5, un clapet anti-retour 51 équipe l'ouverture du conduit d'aspiration 27 située dans la première chambre 320, de manière à éviter tout reflux du bol alimentaire situé dans la première chambre 320, vers le collecteur 2. De même, un clapet anti-retour 53 équipe le conduit de refoulement 29, de manière à éviter tout reflux du bol alimentaire situé dans le tube de refoulement 25 vers la première chambre 320.

En référence à la Fig. 5, un capteur de température 73 est disposé sensiblement au niveau du conduit d'aspiration 27. Ce capteur de température 73 permet de détecter la variation de température de l'intérieur du conduit d'aspiration 27. Une élévation de température correspond à la présence de bol alimentaire dans le conduit 27 ou en amont de ce dernier. Un capteur de pression 75 est disposé dans la deuxième chambre 340 et permet de détecter la variation de la pression de l'air contenu dans la deuxième chambre 340. Le capteur de température 73 et le capteur de pression 75 font partie des moyens de commande 5.

Les moyens de commande sont similaires à ceux décrits en référence au premier mode de réalisation.

Le fonctionnement de ce deuxième mode de réalisation va être décrit plus en détails en référence aux Fig. 4 et 5.

On effectue tout d'abord un étalonnage du dispositif, avant son fonctionnement. Le capteur de température 73 mesure la température de l'air remplissant le conduit d'aspiration 27. Cette valeur correspond à la valeur de référence du dispositif « à vide », c'est-à-dire ne contenant pas de bol alimentaire. De même, le capteur de pression 75 mesure la pression de l'air dans la deuxième chambre 340. La valeur de pression mesurée correspond à la valeur de référence du dispositif « à vide ».

Les capteurs 73 et 75 mesurent ensuite régulièrement, respectivement, la température du milieu contenu dans le conduit d'aspiration 27 et la pression de l'air dans la deuxième chambre 340. La durée entre deux mesures de température et de pression correspond à une durée test, réglable par l'utilisateur via les moyens de commande (la tablette bluetooth®, par exemple). Lorsque du fait des contractions de la portion amont I1 de l'intestin, du bol alimentaire sort par la stomie amont S1, le bol alimentaire pénètre dans le collecteur 2 et parvient dans la section du conduit d'aspiration 27 située au niveau du capteur de température 73. Le bol alimentaire remplit ensuite la première chambre 320 jusqu'à exercer une pression qui déforme la membrane 351. Les capteurs 73 et 75 continuent d'effectuer leurs mesures respectives. Une de ces mesures est effectuée lorsque le bol alimentaire remplit le conduit d'aspiration 27 et entre dans la première chambre 320, déformant la membrane 351. Le capteur 73 mesure la température, la compare avec ladite valeur de référence et détecte la présence de bol alimentaire. Le capteur de pression 75 est une sécurité. Il ne mesure une variation de pression dans la deuxième chambre que lorsque la membrane 351 est bien bombée vers l'intérieur de la deuxième chambre 340, preuve que la première chambre se remplit de plus en plus de bol alimentaire. Lorsque le capteur 75 détecte une augmentation de pression supérieure à un seuil donné, dans la deuxième chambre et que le capteur de température 73 a mesuré une augmentation de température, les moyens de commande actionnent alors les moyens formant pompe 3. La pompe à air 318 injecte de l'air dans la deuxième chambre 340, ce qui a pour effet de courber la membrane 351 vers l'intérieur de la première chambre 320. Le volume interne de cette dernière se réduisant, le bol alimentaire qu'elle contient ferme le clapet anti-retour 51 puis passe à travers le conduit de refoulement 29 et le tube de refoulement 25 Le bol alimentaire est donc ainsi injecté dans la portion aval I2 de l'intestin. La pompe à air 318 aspire alors l'air contenu dans la deuxième chambre 340 ce qui a pour effet de bomber la membrane 351 vers l'intérieur de la deuxième chambre. 340. Le volume de la première chambre 320 augmente ce qui a pour effet de pomper du bol alimentaire contenu dans le collecteur 2, à travers le conduit d'aspiration 27. Les cycles de déformation de la membrane 351 sont effectués jusqu'à ce que le capteur de température ne détecte plus la présence de bol alimentaire, c'est-à-dire jusqu'à ce qu'il détecte une diminution de la température. Les cycles peuvent également être effectués sur une durée donnée programmable via les moyens de commande.

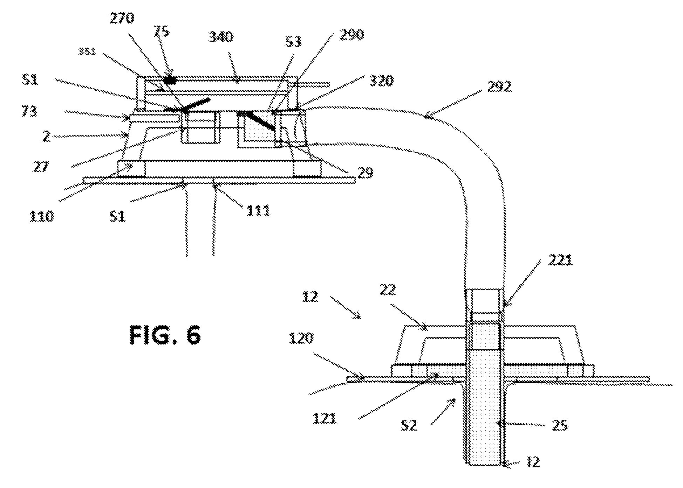

Une variante de réalisation va maintenant être décrite en référence à la Fig. 6. Les éléments en commun avec le deuxième mode de réalisation sont référencés à l'identique. La Fig. 6 correspond à des stomies S1 et S2 éloignées. Dans ce cas, les premiers moyens de connexion étanche 11 comportent un collecteur 2 surmonté de la pompe à membrane ménagée dans le carter 35. La différence avec le mode de réalisation représenté sur la Fig. 5 est que le conduit de refoulement 29 traverse la paroi du collecteur 2 et se prolonge par un tube de liaison souple 292. Ce tube de liaison 292 est relié à un second collecteur 22 équipant les deuxièmes moyens de connexion étanche 12. Les deuxièmes moyens de connexion étanche 12 comportent un support 120 identique au support 110 sur lequel est monté le collecteur 22. Le collecteur 22 comporte une tubulure rigide 221 sur une extrémité de laquelle est monté le tube de liaison 292. La tubulure rigide 221 présente une deuxième extrémité disposée dans le collecteur 22. Le tube de refoulement 25 est monté sur cette deuxième extrémité de la tubulure 221, à l'intérieur du collecteur 22 et prolonge donc le tube de liaison 292. Le tube de refoulement 25 traverse le passage 121, ménagé dans le support 120 et disposé en regard de la stomie aval S2, de manière à pénétrer dans la portion I2 de l'intestin, à travers la stomie aval S2.

Selon une variante non représentée, la pompe à membrane comporte une membrane qui est déformée localement par des doigts mécaniques situés dans la deuxième chambre du carter. Ces doigts mécaniques sont au nombre de trois. Un doigt est disposé dans la deuxième chambre au-dessus de l'ouverture par laquelle débouche le conduit d'aspiration. Un deuxième doigt est disposé dans la deuxième chambre au niveau de l'ouverture par laquelle débouche le conduit de refoulement. Un troisième doigt est disposé entre les deux autres doigts. Les doigts peuvent être rapprochés ou éloignés de la membrane. Lorsqu'ils touchent la membrane, il la déforme de manière élastique. Le fonctionnement de cette variante est le suivant : pour aspirer le bol alimentaire contenu dans le collecteur, le second doigt est activé de manière à plaquer la membrane contre l'ouverture par laquelle débouche le conduit de refoulement, de manière à boucher ce dernier. Le bol alimentaire pénètre dans la première chambre. Le premier doigt est actionné pour venir boucher le conduit d'aspiration. Le troisième doigt est alors activé pour venir presser la membrane de manière à pousser le bol alimentaire contenu dans la première chambre vers le conduit de refoulement. Simultanément, le deuxième doigt est éloigné du conduit de refoulement de manière à laisser le passage du bol alimentaire. Les doigts précités permettent de ne pas utiliser de pompe à air et font à la fois fonction de clapet anti-retour et de moyens formant pompe.

## Revendications

1. Dispositif permettant de créer un flux de bol alimentaire, comportant des moyens formant pompe (3) qui présentent une ouverture d'aspiration (270) et une ouverture de refoulement (290) et qui sont adaptés pour aspirer un bol alimentaire à travers ladite ouverture d'aspiration et le refouler à travers ladite ouverture de refoulement, **caractérisé en ce qu'**il comporte, en outre :
- des premiers moyens de connexion étanche (11) aptes à relier ladite ouverture d'aspiration à une stomie amont (S1) située sur la paroi abdominale d'un patient ;
- des deuxièmes moyens de connexion étanche (12) aptes à relier ladite ouverture de refoulement à une stomie aval (S2), disposée sur la paroi abdominale dudit patient ;
et **en ce que** lesdits moyens formant pompe (3) sont actionnables par un utilisateur et sont aptes à être montés sur le corps dudit patient, notamment au niveau de son abdomen, moyennant quoi lorsque le dispositif est monté sur ledit patient, du bol alimentaire peut circuler de ladite stomie amont (S1) vers ladite stomie aval (S2), à l'extérieur de l'abdomen dudit patient.

2. Dispositif selon la revendication 1, **caractérisé en ce que** lesdits premiers moyens de connexion étanche (11) et/ou lesdits deuxièmes moyens de connexion étanche (12) comportent un socle (110 ; 120) sur lequel est monté un collecteur de bol alimentaire (2; 22), **en ce que** ledit socle (110; 120) présente une première face (112) apte à être fixée de manière étanche sur la peau d'un patient et une deuxième face (114) qui comporte des moyens de montage (117, 118, 119) dudit collecteur (2) et **en ce qu'**au moins un passage (111) débouchant dans ledit collecteur (2) est ménagé dans ledit socle (120).

3. Dispositif selon l'une quelconque des revendications 1 ou 2, **caractérisé en ce que** lesdits deuxièmes moyens de connexion étanche (12) comportent un tube de refoulement (25) relié à ladite sortie de refoulement et conformé pour être introduit dans l'intestin d'un patient au niveau de ladite stomie aval (S2).

4. Dispositif selon l'une quelconque des revendications 2 et 3, **caractérisé en ce que** lesdits premiers moyens de connexion étanche (11) et lesdits deuxièmes moyens de connexion étanche (12) comportent un socle commun (120) et un collecteur commun (2).

5. Dispositif selon l'une quelconque des revendications précédentes, **caractérisé en ce que** lesdits moyens formant pompe comportent au moins un élément déformable (31 ; 351) et des moyens de déformation dudit élément déformable (32, 33 ; 318, 319), **en ce que** ledit élément déformable (31 ; 351) définit au moins partiellement un espace communiquant avec lesdits premiers moyens de connexion étanche (11) au niveau de ladite ouverture d'aspiration et communiquant avec lesdits deuxièmes moyens de connexion étanche (12) au niveau de ladite ouverture de refoulement, **en ce que** les déformations dudit élément déformable (31 ; 351) modifient le volume dudit espace, moyennant quoi lorsque ledit volume dudit espace diminue le bol alimentaire contenu dans ledit espace est expulsé dans lesdits deuxième moyens de connexion étanche (12) et lorsque ledit volume dudit espace augmente du bol alimentaire provenant desdits premiers moyens de connexion étanche (11) est aspiré dans ledit espace.

6. Dispositif selon la revendication 5, **caractérisé en ce que** ledit élément déformable est une membrane (351), **en ce que** lesdits moyens formant pompe (3) comportent un carter (35) dont l'espace interne est divisé en une première (320) et une deuxième chambre (340) par ladite membrane (351), **en ce que** ladite première chambre (320) communique avec lesdits premiers moyens de connexion étanche (11) et avec lesdits deuxièmes moyens de connexion étanche (12), **en ce que** lesdits moyens formant pompe (3) comportent des moyens d'injection/aspiration d'un fluide (318, 319) qui sont reliés à ladite deuxième chambre (340) et qui sont adaptés pour faire varier la pression dudit fluide dans ladite deuxième chambre (340), moyennant quoi lesdits moyens d'injection/aspiration d'un fluide (318, 319) font varier la courbure de ladite membrane déformable (351) ce qui permet d'aspirer du bol alimentaire dans ladite première chambre (340) ou de l'expulser hors de ladite première chambre (340).

7. Dispositif selon l'une quelconque des revendications précédentes, **caractérisé en ce qu'**il comporte des premiers moyens formant clapet (51) disposés pour éviter le reflux du bol alimentaire aspiré par lesdits moyens formant pompe (3) vers lesdits premiers moyens de connexion étanche (11) et/ou des deuxièmes moyens formant clapet (53) pour éviter le reflux du bol alimentaire refoulé par lesdits moyens formant pompe (3) vers lesdits deuxième moyens de connexion étanche (12).

8. Dispositif selon l'une quelconque des revendications précédentes, **caractérisé en ce qu'**il comporte des moyens de commande (5) desdits moyens formant pompe (3), **en ce que** lesdits moyens de commande comportent une horloge interne temps-réel adaptée pour actionner lesdits moyens formant pompe au bout d'une durée de repos donnée, choisie par l'utilisateur et des moyens de programmation de ladite horloge interne temps réel qui permettent de choisir ladite durée de repos.

9. Dispositif selon la revendication 8, **caractérisé en ce que** lesdits moyens de commande (5) comportent, en outre :
- des moyens de détection de la présence du bol alimentaire (71 ; 73 ; 75) en aval desdits premiers moyens de connexion étanche et/ou en amont desdits deuxième moyens de connexion étanche qui sont adaptés pour mesurer un paramètre variable en fonction de la présence ou non de bol alimentaire et actionner lesdits moyens formant pompe (3) lorsque la valeur dudit paramètre correspond à la présence de bol alimentaire dans ledit dispositif ;
- des moyens de déclenchement desdits moyens de détection qui actionnent lesdites moyens de détection au bout d'une durée test choisie par l'utilisateur ; et
- des moyens de réglage de ladite durée test.

10. Dispositif selon la revendication 9, **caractérisé en ce que** lesdits moyens de détection comportent au moins un capteur (71 ; 73 ; 75) choisi parmi les capteurs capacitifs, les capteurs optiques, les capteurs de température et les capteurs de pression.

## Patentansprüche

1. Vorrichtung, die es ermöglicht, einen Flusses eines Speisebreis zu erzeugen, wobei sie Mittel umfasst, die eine Pump (3) bilden, die eine Ansaugöffnung (270) und eine Austrittsöffnung (290) darstellen und die eingerichtet sind, einen Speisebrei durch die Ansaugöffnung zu saugen und durch die Austrittsöffnung zu fördern, **dadurch gekennzeichnet, dass** sie ferner umfasst:
- erste Mittel zur dichten Verbindung (11), die eingerichtet sind, die Ansaugöffnung mit einem stromaufwärtigen Stoma (S1) zu verbinden, das sich an der Bauchwand des Patienten befindet;
- zweite Mittel zur dichten Verbindung (12), die eingerichtet sind, die Austrittsöffnung mit einem stromabwärtigen Stoma (S2) zu verbinden, das sich an der Bauchwand des Patienten befindet;
und dass die Mittel, die die Pumpe (3) bilden, durch einen Benutzer betätigbar sind, und eingerichtet sind, um am Körper des Patienten, insbesondere an seinem Bauch, angebracht zu werden, wodurch, wenn die Vorrichtung an dem Patienten angebracht ist, der Speisebrei von dem stromaufwärtigen Stoma (S1) zu dem stromabwärtigen Stoma (S2) außerhalb des Abdomens des Patienten zirkulieren kann.

2. Vorrichtung nach Anspruch 1, **dadurch gekennzeichnet, dass** die ersten Mittel zur dichten Verbindung (11) und / oder die zweiten Mittel zur dichten Verbindung (12) einen Sockel (110; 120) umfassen, auf dem ein Verteiler für den Speisebrei (2; 22) angebracht ist, dass der Sockel (110; 120) eine erste Fläche (112), die eingerichtet ist, dicht an die Haut eines Patienten befestigt zu werden, und eine zweite Fläche (114) aufweist, die Mittel zur Befestigung (117, 118, 119) des Verteilers (2) umfasst, und dass mindestens ein Durchgang (111), der in dem Kollektor (2) mündet, in dem Sockel (120) ausgebildet ist.

3. Vorrichtung nach einem der Ansprüche 1 oder 2, **dadurch gekennzeichnet, dass** die zweiten Mittel zur dichten Verbindung (12) ein Austrittsrohr (25) umfassen, das mit der Austrittsöffnung verbunden ist, und angepasst ist, um in den Darm von einem Patient bei dem stromabwärtigen Stoma (S2) eingeführt zu werden.

4. Vorrichtung nach einem der Ansprüche 2 und 3, **dadurch gekennzeichnet, dass** die ersten Mittel zur dichten Verbindung (11) und die zweiten Mittel zur dichten Verbindung (12) einen gemeinsamen Sockel (120) und einen gemeinsamen Verteiler (2) umfassen.

5. Vorrichtung nach einem der vorhergehenden Ansprüche, **dadurch gekennzeichnet, dass** die Mittel, die die Pumpe bilden, mindestens ein verformbares Element (31; 351) und Mittel zum Verformen des verformbaren Elements (32, 33; 318, 319) aufweisen, dass das verformbare Element (31; 351) zumindest teilweise einen Raum, der mit den ersten Mitteln zur dichten Verbindung (11) bei der Ansaugöffnung verbunden ist und mit dem zweiten Mitteln zur dichten Verbindung (12) bei der Austrittsöffnung verbunden ist, definiert, dass die Verformungen des verformbaren Elements (31; 351) das Volumen des Raumes verändern, wodurch, wenn sich das Volumen des Raums verkleinert, der Speisebrei, der in dem Raum enthalten ist, in die zweiten Mitteln zur dichten Verbindung (12) ausgestoßen wird, und wenn sich das Volumen des Raums vergrößert, der Speisebrei, der von den ersten Mitteln zur dichten Verbindung (11) kommt, in den Raum angesaugt wird.

6. Vorrichtung nach Anspruch 5, **dadurch gekennzeichnet, dass** das verformbare Element eine Membran (351) ist, dass die Mittel, die die Pumpe (3) bilden, ein Gehäuse (35) umfassen, dessen Innenraum in eine erste (320) und eine zweite (340) Kammer durch die Membran (351) unterteilt ist, dass die erste Kammer (320) mit den ersten Mitteln zur dichten Verbindung (11) und mit dem zweiten Mitteln zur dichten Verbindung (12) verbunden ist, dass die Mittel, die die Pumpe (3) bilden, Mittel zum Injizieren / Ansaugen einer Flüssigkeit (318, 319) umfassen, die mit der zweiten Kammer verbunden sind und die eingerichtet sind, den Druck des Fluids in der zweiten Kammer (340) zu variieren, wodurch die Mittel zum Injizieren / Ansaugen einer Flüssigkeit (318, 319) bewirken, dass die Krümmung der verformbaren Membran (351) variiert wird, was das Ansaugen des Speisebreis in der ersten Kammer (340) oder das Ausstoßen aus der ersten Kammer (340) ermöglicht.

7. Vorrichtung nach einem der vorhergehenden Ansprüche, **dadurch gekennzeichnet, dass** sie erste Mittel, die ein Ventil (51) bilden und angebracht sind, um den Rückfluss des Speisebreis, der durch die Mittel, die die Pumpe (3) bilden, gefördert wird, zu den ersten Mitteln zur dichten Verbindung (11) zu verhindern, und / oder zweite Mittel aufweist, die ein Ventil (53) bilden, um den Rückfluss des Speisebreis, der durch die Mittel, die die Pumpe (3) bilden, gefördert wird, zu den zweiten Mitteln zur dichten Verbindung (12) zu verhindern,.

8. Vorrichtung nach einem der vorhergehenden Ansprüche, **dadurch gekennzeichnet, dass** sie Mittel zur Steuerung (5) der Mittel, die die Pumpe (3) bilden, umfasst, dass die Mittel zur Steuerung eine interne Echtzeituhr umfassen, die eingerichtet ist, die Mittel, die die Pumpe bilden, am Ende einer vorgegeben Erholungszeit, die vom Benutzer gewählt wird, und die Mittel zur Programmierung der internen Echtzeituhr zu betätigen, die es ermöglichen, um die Länge der Erholungszeit auszuwählen.

9. Vorrichtung nach Anspruch 8, **dadurch gekennzeichnet, dass** die Mittel zur Steuerung (5) ferner umfassen:
- Mittel zur Erfassung des Vorhandenseins des Speisebreis (71; 73; 75) stromabwärts von dem ersten Mitteln zur dichten Verbindung und / oder stromaufwärts von den zweiten Mitteln zur dichten Verbindung, die eingerichtet sind, einen variablen Parameter für die Messung als Funktion von dem Vorhandensein oder Nichtvorhandensein von Speisebrei zu messen und die Mittel, die die Pumpe (3) bilden, zu betätigen, wenn der Wert des Parameters dem Vorhandensein von Speisebrei in der Vorrichtung entspricht;
- Mittel zum Auslösen der Mittel zur Erfassung, welche die Mittel zur Erfassung am Ende einer Testdauer auslösen, die von dem Benutzer ausgewählt ist; und
- Mitteln zum Regeln der Testdauer.

10. Vorrichtung nach Anspruch 9, **dadurch gekennzeichnet, dass** die Mittel zur Erfassung mindestens einen Sensor (71; 73; 75) umfassen, der aus mindestens den kapazitiven Sensoren, den optischen Sensoren, den Temperatursensoren und den Drucksensoren ausgewählt ist.

## Claims

1. Device enabling food bolus flow between two stomas, said device comprising pump forming means (3), said pump forming means (3) having an entry opening and an exit opening and being suitable for sucking food bolus through said entry opening and releasing it through said exit opening, wherein said device further comprises
- first watertight connection means (11) suitable for connecting said entry opening to a upstream stoma (S1) located on a patient's abdominal wall ;
- second watertight connection means (1) suitable for connecting said exit opening to a downstream stoma (S2), located on said patient's abdominal wall;
said pump forming means being able to be activated by a user and suitable for being mounted on said patient's body, particularly on said patient's abdomen, in such a manner that once said device is mounted onto said patient's abdomen, food bolus flow can circulate from said upstream stoma (S1) to said downstream stoma (S2) outside of said patient's abdomen.

2. Device according to claim 1, wherein first watertight connection means and/or said second watertight connection means (11 ; 12) comprise a base (110 ; 120) onto which a food bolus collector (2 ; 22) is mounted, said base (110 ; 120) having a first surface (112) adapted for being fixed in a watertight manner to a patient's skin, said base (110 ; 120) further having a second surface (114) comprising means for mounting (117 ; 118 ; 119) said connector (2 ; 22) and at least one passage (111) provided within said base (120) and opening into said collector (2).

3. A device according to claim 1 or 2, wherein second watertight connection means (12) comprise an exit flow tube (25) connected to said exit opening and adapted to be introduced into a patient's intestine through said downstream stoma (S2).

4. Device according to any one of claims 2 and 3, wherein said first watertight connection means (11) and said second watertight connection means (12) comprise a common base (120) and a common food bolus connector (2).

5. A device according to any one of claims 1 to 4, wherein said pump forming means (3) comprise at least one deformable part (31 ; 351) and means for deforming said deformable part (32 ; 33; 318 ; 319), in that said deformable part (31 ; 351) defines at least partially a space communicating with said first watertight connection means (11) through said entry opening and communicating with said second watertight connection means (12) through said exit opening, in that deformation of said deformable part (31 ; 351) changes the volume of said space, with the result that when said volume of said space is reduced, food bolus inside said space is expressed into said second watertight connection means (12), and when the volume of said space increases, food bolus coming from said first watertight connection means (11) is sucked into said space.

6. Device according to claim 5, wherein said deformable part is a membrane (351) and said pump forming means (3) comprise a casing (35), the internal space thereof is divided into a first (320) and second chamber (34) by said membrane (351), in that said first chamber (320) communicates with said first watertight connection means (11) and said second watertight connection means (12), in that said pump forming means (3) further comprise means for sucking/releasing a fluid (318; 319), which are connected to said second chamber (340) and which are able of varying the pressure of said fluid within said second chamber (340), with the result that said means for sucking/releasing a fluid (318 ; 319) vary the curve of said deformable membrane (351), therefore enabling food bolus to be sucked into said first chamber (320) or released from said first chamber (320).

7. Device according to any one of the above-mentioned claims, wherein said device further comprises first valve forming means (51) provided for avoiding reflux of food bolus sucked by said pump forming means (3) towards said first watertight connection means (11) and/or second valve forming means (53) provided for avoiding reflux of food bolus released by said pump forming means (3) towards said second watertight connection means (12).

8. Device according to any one of the above-mentioned claims, wherein said device further comprises control means (5) for controlling said pump forming means, in that said control means (5) comprise an internal real time clock adapted for activating said pump forming means after a given rest period chosen by the user and means for programming said real time internal clock provided for selecting said rest period.

9. Device according to claim 8, wherein said control means (5) further comprises
- detection means (71 ; 73 ; 75) for detecting the presence of food bolus downstream to said first watertight connection means (11) and/or upstream to said second watertight connection means (12), said detection means (71 ; 73 ; 75) being able to measure a parameter depending on food bolus presence or absence and to activate said pump forming means (3) once the value of said parameter corresponds to the presence of food bolus inside said device;
- means for triggering said detection means, which activate said detection means after a test period selected by the user; and
- means for adjusting said test period.

10. Device according to claim 9, wherein said detection means (71; 73 ; 75) comprise at least one sensor selected from capacitive sensors, optical sensors, temperature sensors and pressure sensors.
